# EUROPEAN PATENT APPLICATION

(11) **EP 4 449 995 A1**
(43) Date of publication of application: **23.10.2024**
(21) Application number: 24166151.1
(22) Date of filing: 26.03.2024
(51) Int. Cl.: A61B 6/03, A61B 6/46

(54) **IMAGE DISPLAY PRESET SYSTEM AND METHOD FOR C-ARM IMAGING SYSTEM**

(30) Priority: 17.04.2023 US 202318135482
(71) Applicant: GE Precision Healthcare LLC, Waukesha, WI 53188 (US)
(72) Inventor: HERNANDEZ, Heber, Salt Lake City, 84105 (US); BROWN, Jordan, Woods Cross, 84087 (US)
(74) Representative: Fennell, Gareth Charles

(57) **Abstract**

A C-arm x-ray imaging device (100) includes a radiation source (120), a detector (122), a control mechanism (132) and a computing device (144) to control an imaging procedure performed by the imaging system (100). The computing device (144) includes a processor (146) and an interconnected database (148) containing machine-readable instructions for the operation of the processor (146) and for processing the image data from the detector (122) to create one or more 2D images of a subject (118) and to reconstruct a 3D volume (164) from the one or more 2D images. The processor (146) is configured to determine the distribution of radiation attenuation values (172) across at least one portion (166) of the 3D volume (164), to determine a window preset (192,194) for different material types represented in the distribution of radiation attenuation values (172), where the window presets (192,194) are presented along with the 2D/3D images on the device display (150) to allow quick switching between images optimizing viewing of different types of materials within the images.

## Description

### BACKGROUND OF DISCLOSURE

The subject matter disclosed herein relates to radiography imaging systems having C-arms and, more particularly, to display systems and methods for presenting the images obtained by the C-arm radiography imaging systems.

Medical diagnostic imaging systems generate images of an object, such as a patient, for example, through exposure to an energy or radiation source, such as X-rays passing through a patient, for example. The generated images may be used for many purposes. Often, when a practitioner takes X-rays of a patient, it is desirable to take several X-rays of one or more portions of the patient's body from a number of different positions and angles, and preferably without needing to frequently reposition the patient. To meet this need, C-arm X-ray diagnostic equipment has been developed. The term C-arm generally refers to an radiography imaging device having a rigid and/or articulating structural member having a radiation source, such as an X-ray tube, and an image detector assembly that are each located at an opposing end of the structural member so that the radiation source and the image detector face each other. The structural member is typically "C" shaped and so is referred to as a C-arm. In this manner, as an example, X-rays emitted from the X-ray tube functioning as the radiation source can impinge on the image detector and provide X-ray image data/X-ray images of the object or objects that are placed between the X-ray tube and the image detector.

In many cases, C-arms are connected to one end of a movable arm disposed on a base or gantry. In such cases, the C-arm can often be raised and lowered, be moved from side to side, and/or be rotated about one or more axes of rotation via the moveable arm. Accordingly, such C-arms can be moved and reoriented to allow X-ray images to be taken from several different positions and angles and different portions of a patient, without requiring the patient to be frequently repositioned.

The processes that can be performed by the radiography imaging system on the image data obtained by the operation of the radiation source and detector enable a variety of different images to be presented on a display for the radiography imaging system. The processes, which include but are not limited to, cone beam computed tomography, (CBCT), can be employed to reconstruct a number of 2D images taken at various angles relative to the object being imaged into a 3D image of the object. The 3D image or volume can subsequently be processed to provide an image or slice on the display of a selected area or type of tissue present within the imaged object for review and analysis by the imaging system and/or the physician.

However, while certain types of tissue in an imaged object, such as bone tissue, can be readily presented in images provided from a CBCT imaging process, the limitations of the power output of mobile C-arm imaging systems and the lack of strong contrast for other types of tissue present within the 2D image(s) and reconstructed 3D volume create issues when attempting to present images displaying those types of tissues, e.g., lung tissue. As a result, when trying to provide images of these types of tissues, e.g., lung tissue, an operator of the mobile C-arm radiography imaging system must manually adjust the setting for the display of the desired images in order to accommodate for these issues to present an image the adequately represents the desired tissue type with the suitable contrast in the image for viewing of the tissue for diagnostic purposes. As this manual process necessarily requires significant time, effort and experience on the part of the mobile C-arm radiography system operator in order to produce images useable for diagnostic purposes, the manual process presents a significant limitation regarding the quality and speed of production of diagnostic quality images.

Therefore, it is desirable to develop an image processing system and method for a mobile C-ram radiography imaging system that provide an improved manner of producing or slices for optimal viewing of tissues without the need for operator input.

### BRIEF DESCRIPTION OF THE DISCLOSURE

According to one exemplary non-limiting aspect of the disclosure, a radiography imaging device includes a radiation source, a detector alignable with the radiation source, the detector having a support on or against which a subject to be imaged is adapted to be positioned, a computing device operably connected to the detector to generate image data in an imaging procedure performed by the imaging system, the computing device including a processor and an interconnected database containing machine-readable instructions for the operation of the processor and for processing the image data from the detector to create one or more 2D images of an subject and to reconstruct a 3D volume from the one or more 2D images, a display operably connected to the computing device for presenting the one or more 2D images, the 3D volume or one or more portions thereof, and combination thereof to a user and a user interface operably connected to the computing device to enable user input to the control processing unit, wherein the processor is configured to determine the distribution of radiation attenuation values across at least one portion of the 3D volume, to determine a first window level and a first window width for a first material type represented in the distribution of radiation attenuation values to form a first window preset; and to determine a second window level and a second window width for the second material type represented in the distribution of radiation attenuation values to form a second window preset.

According to still another aspect of one exemplary non-limiting embodiment of the disclosure, a method for adjusting a presentation of an image presented on a display of a radiography imaging system, having the steps of providing an imaging system including a radiation source, a detector alignable with the radiation source, the detector having a support on or against which a subject to be imaged is adapted to be positioned, a computing device operably connected to the detector to generate image data in an imaging procedure performed by the imaging system, the computing device including a processor and an interconnected database containing machine-readable instructions for the operation of the processor and for processing the image data from the detector to create one or more 2D images of an subject, a display operably connected to the computing device for presenting the one or more 2D images to a user, and a user interface operably connected to the computing device to enable user input to the control processing unit, positioning the subject between the radiation source and the detector, operating the x-ray source to generate a plurality of projection images of the subject, reconstructing a 3D volume from the plurality of projection images, determining a distribution of radiation attenuation values from at least one portion of the 3D volume, and determining a window preset from the distribution of radiation attenuation values corresponding to each type of material represented in the distribution of radiation attenuation values.

It should be understood that the brief description above is provided to introduce in simplified form a selection of concepts that are further described in the detailed description.

### BRIEF DESCRIPTION OF THE DRAWINGS

The drawings illustrate the best mode presently contemplated of carrying out the disclosure. In the drawings:
FIG. 1 is a side elevation view of an embodiment of a radiography medical imaging device according to one non-limiting exemplary embodiment of the disclosure.
FIG. 2 is a block diagram of a control mechanism for the radiography medical imaging device of FIG. 1 according to one exemplary non-limiting embodiment of the disclosure.
FIG. 3 is a block diagram of a computing device for the radiography medical imaging device of FIG. 1 according to one exemplary non-limiting embodiment of the disclosure.
FIG. 4 is a flowchart of a method of operation of the radiography medical imaging device of FIG. 1 according to one exemplary non-limiting embodiment of the disclosure.
FIG. 5 is a top plan view of a slice of a reconstructed 3D volume formed by the radiography medical imaging device of FIG. 1 according to one exemplary non-limiting embodiment of the disclosure.
FIG. 6 is a graph of the number of pixels in the slice of FIG. 5 having specified radiation attenuation values and identifying different types of materials present in the slice according to another exemplary embodiment of the disclosure.
FIG. 7 is graph representing the determination of a bone window level and width and a lung window level and width using the graph of FIG. 6 according to another exemplary embodiment of the disclosure.
FIG. 8 is a schematic view of the training and/or operation of a window generating module forming a part of the computing device of FIG. 3 according to another exemplary embodiment of the disclosure.
FIG. 9 is a pictoral view of a display of the medical imaging device of FIG. 1 illustrating images utilizing a bone window preset and including a lung window preset icon according to another exemplary embodiment of the disclosure.
FIG. 10 is a pictoral view of a display of the radiography medical imaging device of FIG. 1 illustrating images utilizing a lung window preset and including a bone window preset icon according to another exemplary embodiment of the disclosure.
FIGS. 11A-11B are pictoral views of display of the radiography medical imaging device of FIG. 1 illustrating images utilizing a bone window preset thickening algorithm and a lung window preset thickening algorithm according to another exemplary embodiment of the disclosure.

### DETAILED DESCRIPTION

One or more specific embodiments will be described below. In an effort to provide a concise description of these embodiments, all features of an actual implementation may not be described in the specification. It should be appreciated that in the development of any such actual implementation, as in any engineering or design project, numerous implementation-specific decisions must be made to achieve the developers' specific goals, such as compliance with system-related and business-related constraints, which may vary from one implementation to another. Moreover, it should be appreciated that such a development effort might be complex and time consuming, but would nevertheless be a routine undertaking of design, fabrication, and manufacture for those of ordinary skill having the benefit of this disclosure.

When introducing elements of various embodiments of the present disclosure, the articles "a," "an," and "the" are intended to mean that there are one or more of the elements. The terms "first," "second," and the like, do not denote any order, quantity, or importance, but rather are used to distinguish one element from another. The terms "comprising," "including," and "having" are intended to be inclusive and mean that there may be additional elements other than the listed elements. As the terms "connected to," "coupled to," etc. are used herein, one object (i.e., a material, element, structure, number, etc.) can be connected to or coupled to another object regardless of whether the one object is directly connected or coupled to the other object or whether there are one or more intervening objects between the one object and the other object. In addition, it should be understood that references to "one embodiment" or "an embodiment" of the present disclosure are not intended to be interpreted as excluding the existence of additional embodiments that also incorporate the recited features.

As used herein, "electrically coupled", "electrically connected", and "electrical communication" mean that the referenced elements are directly or indirectly connected such that an electrical current may flow from one to the other. The connection may include a direct conductive connection, i.e., without an intervening capacitive, inductive or active element, an inductive connection, a capacitive connection, and/or any other suitable electrical connection. Intervening components may be present. (2888.142)

Certain examples provide an image processing apparatus/window generation module including an artificial intelligence system (AI system). The AI system can analyze a reconstructed 3D volume and/or 3D volume dataset in order to determine a range of attenuation values for the object being imaged and provide one or more settings for the windows to display 2D/3D images of the imaged object with optimized brightness and contrast for the content within the image, for example. The AI system can be a discrete output of positive or negative for a finding, a segmentation, etc. For example, the AI system can instantiate machine learning and/or other artificial intelligence to detect, and analyze the attenuation values present within a 3D volume/3D volume dataset provided to the AI system. For example, the AI system can instantiate machine learning and/or other artificial intelligence to detect the attenuation within a 3D volume/3D volume dataset, or portion(s) thereof, provided by a detector operably connected to the imaging system, to differentiate the number and/or type of materials and/or tissues present within the 3D volume/3D volume dataset, or portions thereof, to determine the window level and width settings for the optimized viewing of each typer of material and/or tissue present within the 3D volume/3D volume dataset, or portions thereof, and to provide one or more image presets for 2D/3D images generated from the 3D volume/3D volume dataset, or portions thereof, to maximize the visibility of each of the various materials and/or tissues within the 2D/3D image.

Machine learning techniques, whether deep learning networks or other experiential/observational learning system, can be used to locate an object in an image, understand speech and convert speech into text, and improve the relevance of search engine results, for example. Deep learning is a subset of machine learning that uses a set of algorithms to model high-level abstractions in data using a deep graph with multiple processing layers including linear and non-linear transformations. While many machine learning systems are seeded with initial features and/or network weights to be modified through learning and updating of the machine learning network, a deep learning network trains itself to identify "good" features for analysis. Using a multilayered architecture, machines employing deep learning techniques can process raw data better than machines using conventional machine learning techniques. Examining data for groups of highly correlated values or distinctive themes is facilitated using different layers of evaluation or abstraction.

Throughout the specification and claims, the following terms take the meanings explicitly associated herein, unless the context clearly dictates otherwise. The term "deep learning" is a machine learning technique that utilizes multiple data processing layers to recognize various structures in data sets and classify the data sets with high accuracy. A deep learning network can be a training network (e.g., a training network model or device) that learns patterns based on a plurality of inputs and outputs. A deep learning network can be a deployed network (e.g., a deployed network model or device) that is generated from the training network and provides an output in response to an input.

The term "supervised learning" is a deep learning training method in which the machine is provided already classified data from human sources. The term "unsupervised learning" is a deep learning training method in which the machine is not given already classified data but makes the machine useful for abnormality detection. The term "semi-supervised learning" is a deep learning training method in which the machine is provided a small amount of classified data from human sources compared to a larger amount of unclassified data available to the machine. Certain examples use neural networks and/or other machine learning to implement a new workflow for image and associated patient analysis including automated alteration of the display of images and associated information generated and delivered at the point of care of a radiology exam. Certain examples use Artificial Intelligence (AI) algorithms to process a 3D volume/3D volume dataset obtained during one or more imaging exams (e.g., an image or set of images), and provide one or more image presets including the window level and the window width for the optimized display of a 2D/3D image including a detected type of material and/or tissue within the 2D/3D image being displayed. The image preset(s) (e.g., window level, window width, brightness, contrast, etc.) may be intended for the technologist acquiring the exam, clinical team providers (e.g., nurse, doctor, etc.), radiologist, administration, operations, and/or even the patient. The image presets can be presented on the display along with the 2D/3D image to enable rapid switching between views of the presented 2D/3D image in order to highlight or render more visible different types of materials and/or tissues present within the displayed 2D/3D image.

In certain examples, the AI algorithm can be (1) embedded within an imaging device, (2) running on a mobile device (e.g., a tablet, smart phone, laptop, other handheld or mobile computing device, etc.), and/or (3) running in a cloud (e.g., on premise or off premise) and delivers the alert via a web browser (e.g., which may appear on the radiology system, mobile device, computer, etc.). Such configurations can be vendor neutral and compatible with legacy imaging systems. For example, if the AI processor is running on a mobile device and/or in the "cloud", the configuration can receive the images (A) from the x-ray and/or other imaging system directly (e.g., set up as secondary push destination such as a Digital Imaging and Communications in Medicine (DICOM) node, etc.), (B) by tapping into a Picture Archiving and Communication System (PACS) destination for redundant image access, (C) by retrieving image data via a sniffer methodology (e.g., to pull a DICOM image off the system once it is generated), etc.

Certain examples provide apparatus, systems, methods, etc., to provide one or more image presets for 2D/3D image to be presented on a display based on output of an algorithm instantiated using and/or driven by an artificial intelligence (AI) model, such as a deep learning network model, machine learning network model, etc. For example, the image presets are each configured to provide an optimized brightness and/or contrast for a detected type of material and/or tissue within the 2D/3D image to be presented on the display based on an output of an AI algorithm.

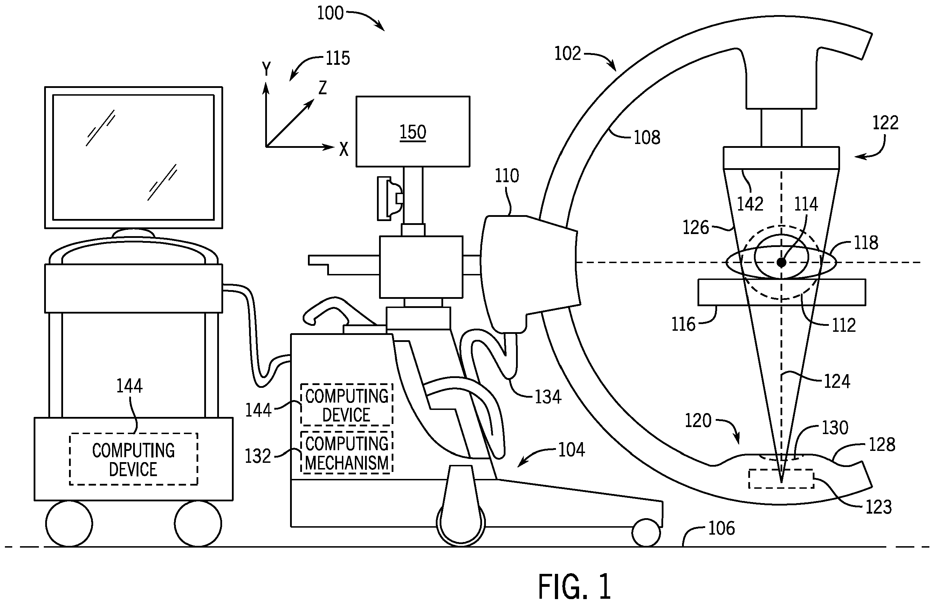

Medical imaging systems may include a C-shaped arm that carries a radiation source and a radiation detector. The C-shape of the arm allows a physician to access to a patient while the patient is being imaged. In order to obtain medical images of an internal structure at various angles, the C-shaped arm may be rotated to various positions. The following description relates to various embodiments for a medical imaging system with a C-arm. A medical imaging system, such as the medical imaging system shown in FIG. 1 , includes a C-arm configured to rotate around at least one rotational axis. The C-arm includes a radiation source and a radiation detector at opposite ends of the C-arm.

Referring to the figures generally, the present disclosure describes systems and methods for a medical imaging system with a C-arm. The medical imaging system described herein (i.e., the medical imaging system depicted in FIG. 1) may be generally referred to as a radiographic medical imaging system, and in particular a mobile C-arm radiographic or radiography imaging system.

Referring now to FIG. 1, a medical radiography imaging device or system 100, such as that disclosed in US Published Patent Application Serial No. US2022/0401048, entitled *Imaging System With Carbon Fiber C-Arm*, the entirety of which is expressly incorporated herein by reference for all purposes, is shown in accordance with an exemplary embodiment. The medical radiography imaging device or system 100 includes a rotatable C-arm 102 that is connected to a base 104. The base 104 supports the C-arm 102 while the C-arm 102 is stationary and while rotating. The base 104 supports the C-arm 102 on a ground surface 106 on which the medical radiography imaging device or system 100 sits via a number of wheels 105 or similar rotatable supports that enable the base 104 to be readily moved over and/or along the surface 106 by an operator, such as by grasping handles 107 on the base 104 and pulling or pushing the medical radiography imaging device or system 100 into the desired position for the operation of the medical radiography imaging device or system 100. The C-arm 102 includes a C-shaped portion 108 that is connected to an extended portion 110. The extended portion 110 is rotatably coupled to the base 104 which allows the C-arm 102 to rotate about an examination region 112 and a rotational axis 114. For example, the C-arm 102 may be configured to rotate at least 180° in opposing directions relative to the base 104, though in some embodiments, the C-arm 102 may be configured to rotate at least 220°. Configuring the C-arm 102 to rotate at least 220° may provide a physician with greater access to a patient being imaged. While the following describes the rotation of the C-arm 102 as rotating in the X and Y directions of the Cartesian coordinate system 115 (i.e., rotating the C-shaped portion 108 such that opposing ends of the C-shaped portion 108 are closer to or further from the extended portion 110 in various positions), it is understood that the C-arm 102 may also rotate in the Z direction (i.e., rotating the C-shaped portion 108 such that opposing ends of the C-shaped portion 108 are closer to or further from a head of the patient within the examination region 112 in various position and/or changing the elevation of the extended portion 110 relative to the base 104 employing a suitable vertical translation mechanism (not shown) disposed on the base 104 and engaged with the extended portion 110.

The medical radiography imaging device or system 100 further includes a patient support 116 (i.e., couch, bed, table, etc.) that supports an object, subject or patient, such as a patient 118 while at least a portion of the patient 118 is within the examination region 112. The medical radiography imaging system 100 additionally includes a radiation source 120 and a radiation detector 122. The radiation source 120 and the radiation detector 122 are supported by and rotate with the C-arm 102. Furthermore, the radiation source 120 and the radiation detector 122 are positioned at opposite ends of the C-shaped portion 108 of the C-arm 102 along axis 124, where axis 124 intersects and extends radially relative to the rotational axis 114. The C-shaped portion 108 may be rotated as described above in order to adjust the position of the radiation source 120 and the radiation detector 122 to obtain 2D projection images of the subject 118 at each selected angular position or orientation, e.g., two or more angular positions, of the radiation source 120 and the detector 122 relative to the patient 118 in order to form a 2D projection dataset. Furthermore, in the embodiment depicted in FIG. 1, the position of the radiation detector 122 may be varied such that the radiation detector 122 is placed further from or closer to the radiation source 120.

During a medical imaging procedure, a portion of the patient 118 is within the examination region 112 and the radiation source 120 emits radiation 126. In one embodiment, the radiation source 120 may include an X-ray tube 123 housed within a casing 128. The X-ray tube generates the radiation 126 which escapes the casing 128 via an outlet 130. The radiation 126 traverses the examination region 112 and is attenuated by the portion of the patient 118 that is within the examination region 112. Specifically, the radiation source 120 emits the radiation 126 towards the radiation detector 122 which is on the opposite end of the C-arm 102. The radiation source 120 emits cone-shaped radiation which is collimated to lie within an X-Y-Z plane of the Cartesian coordinate system 115 which is generally referred to as an "object plane" which is parallel to the radiation detector 122 at an isocenter of the C-arm 102.

After passing through a portion of the patient 118, the attenuated radiation is captured by the radiation detector 122. In some embodiments, the radiation detector 122 includes a plurality of detector elements (not shown) that acquire projection data. Each detector element produces an electrical signal that is a measurement of the attenuation at the detector element location. The attenuation measurements from all the detector elements in the detector 122 are acquired separately to produce a transmission profile. In one embodiment, the radiation detector 122 is fabricated in a flat panel configuration including a plurality of detector elements.

When the radiation source 120 and the radiation detector 122 are rotated with the C-arm 102 within the object plane and around the patient 118, the angle at which the radiation 126 intersects the patient 118 changes. A group of attenuation measurements (i.e., projection data) form the radiation detector 122 at one C-arm angle is referred to a "view." A "scan" of the patient 118 includes a set of projection views made at different angles, or view angles, during rotation of the C-arm 102. As used herein, the term view is not limited to the use described herein with respect to projection data obtained from or from one C-arm 102 angle. The term view is used to mean one data acquisition whenever there are multiple acquisitions from different angles, such as used to form the 2D projection dataset.

The medical radiography imaging system 100 further includes a control mechanism 132 that is housed within the base 104. The control mechanism 132 is connected to the C-arm 102, the radiation source 120, and the radiation detector 122 via a cable 134 which allows the control mechanism to send data to/receive data from the C-arm 102, the radiation source 120, and the radiation detector 122. The control mechanism 132 controls the rotation of the C-arm 102 and the operation of the radiation source 120.

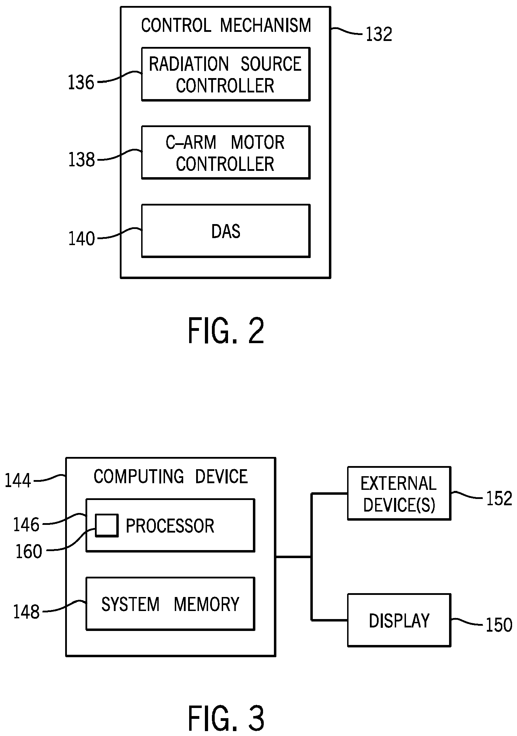

Briefly turning to FIG. 2, a block diagram of the control mechanism 132 is shown in accordance with an exemplary embodiment. In one embodiment, the control mechanism 132 includes a radiation source controller 136 and a C-arm motor controller 138. The radiation source controller 136 is configured to provided power and timing signals to the radiation source 120. The C-arm motor controller 138 is configured to control a rotation speed and/or position of the C-arm 102. Furthermore, the C-arm motor controller controls the rotation axis of the C-arm 102, a position of the detector 122 and thereby controlling a source to detector distance, and a location of the patient support 116.The control mechanism 132 further includes a data acquisition system (DAS) 140. The DAS 140 is configured to sample analog data received from the radiation detector 122 and convert the analog data to digital signals for subsequent processing. While FIG. 1 depicts the base 104 as including the control mechanism 132, in other embodiments the control mechanism may be separate from the base 104 (i.e., in a different room).

The C-arm 102 may be adjusted to a plurality of different positions by rotation of the C-shaped portion 108. For example, in an initial, first position shown by FIG. 1, the radiation detector 122 may be positioned vertically above the radiation source 120 relative to the surface 106 on which the medical radiography imaging system 100 sits, with axis 124 arranged normal to the surface 106 intersecting a midpoint of the outlet 130 of the radiation source 120 and a midpoint of a detector surface 142 of the radiation detector 122. The C-arm motor controller 138 and a guide system within the extended portion 110 may adjust the C-shaped portion 108 from the first position to a different second position by rotating the C-shaped portion 108 via a coupling between the guide system and the C-shaped portion 108. In one example, the second position may be a position in which the radiation source 120 and the detector 122 are rotated 180° together relative to the first position such that the radiation source 120 is positioned vertically above the radiation detector 122, with the axis 124 intersecting the midpoint of the outlet 130 of the radiation source 120 and the midpoint of the detector surface 142 of the radiation detector 122. When adjusted to the second position, the radiation source 120 may be positioned vertically above the rotational axis 114 of the C-shaped portion 108 and the radiation detector 122 may be posited vertically below the rotational axis 114.

The medical radiography imaging device or system 100 further includes a computing device 144 that is housed within the base 104. While FIG. 1 depicts the computing device 144 as housed within the base 104, in other embodiments the computing device 144 may be remote from the rest of the medical radiography imaging device or system 100. As used herein, a computing device (or system) is any device/system capable of processing, storing, and/or transmitting data (i.e., tablet, handheld device, smartphone, personal computer, laptop, network computer, server, mobile communication device, etc.). The computing device 144 may be connected to a network (i.e., a wide area network (WAN), a local area network (LAN), a public network (the internet), etc.) which allows the computing device 144 to communicate with other devices on a same network. In some embodiments, the network may be regarded as a private network and may include, for example, a virtual private network.

Briefly turning to FIG. 3, a block diagram of the computing device 144 is shown in accordance with an exemplary embodiment. The computing device 144 includes a processor 146 and a system memory 148. In some embodiments, the computing device is connected to a display 150 and one or more user input devices, e.g., a touchscreen, a keyboard, a mouse, etc., and/or external devices 152. The processor 146 is in communication with the system memory 148 and may execute computer readable program instructions stored in the system memory 148. As used herein, a processor may include a central processing unit (CPU), or other electronic components capable or executing computer readable program instructions (i.e., a digital signal processor, a field-programmable gate array (FPGA), a graphics processing unit (GPU), etc.). Furthermore, as used herein, a processor may include two or more of a CPU, a digital signal processor, an FPGA, and a GPU.

The system memory 148 is a computer readable storage medium. As used herein, a computer readable storage medium is any device that stores computer readable program instructions for execution by a processor and is not construed as transitory per se. Computer readable program instructions include programs, logic, data structures, modules, etc. that when executed by a processor create a means for implementing functions/acts. Computer readable program instructions when stored in a computer readable storage medium and executed by a processor direct a computer system and/or another device to function in a particular manner such that a computer readable storage medium comprises an article of manufacture. System memory as used herein includes volatile memory (i.e., random access memory (RAM) and dynamic RAM (DRAM)) and non-volatile memory (i.e., flash memory, read-only memory (ROM), magnetic computer storage devices, etc.). In some embodiments the system memory 148 may further include cache.

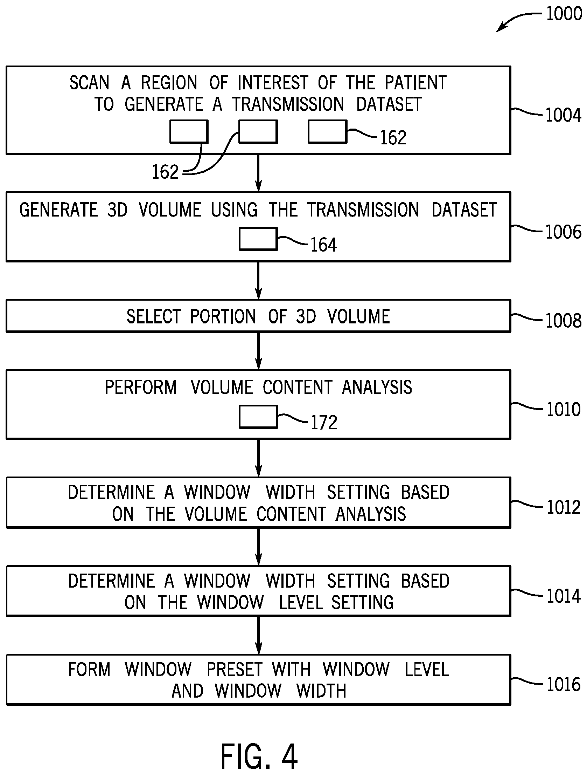

In one embodiment, the various methods and processes (i.e., the method described below with reference to FIG. 4) may be stored as computer readable program instructions in the system memory 148. In this embodiment, the system memory 148 includes computer readable program instructions for imaging a patient with a medical imaging system (i.e., the medical imaging device or system 100).

The external devices 152 include devices that allow a user to interact with/operate the computing device 144 (i.e., mouse, keyboard, touchscreen, speakers, etc.), and can include the display 150 when configured as a touchscreen device. In some embodiments, the display 150 displays a graphical user interface (GUI). The GUI includes editable fields for inputting data (i.e., patient data, imaging parameters, etc.) and further includes selectable icons. Selecting an icon and/or inputting data causes the processor 146 to execute computer readable program instructions stored in the system memory 148 which causes the processor to perform a task. For example, a user of the computing device 144 may use an external device 152 or the touchscreen display 150 to select a "start" icon or the like which causes the processor 146 to being a medical imaging procedure and/or analysis according to one or more embodiments as disclosed herein.

While FIG. 1 illustrates only one computing device 144, in some embodiments, the medical radiography imaging device or system 100 may include more than one computing device 144. The computing device 144 may be used for inputting or outputting imaging parameters, requesting examinations, plotting data, and/or viewing images. Furthermore, in certain embodiments, the medical radiography imaging device or system 100 may be coupled to multiple displays, printers, workstations, and/or similar devices located either locally or remotely (i.e., within an institution or hospital or in a an entirely different location, etc.) via one or more configurable wired and/or wireless networks. Furthermore, in some embodiments, the base further house an internal power source (not shown) that provides electrical power to operate the medical imaging device or system 100. Alternatively, the base 104 may be connected to an external power source to power the medical radiography imaging device or system 100. A plurality of connection cables may (i.e., cable 134) may be provided to transmit electrical power to the radiation source 120, the radiation detector 122, etc.

The computing device 144 is in communication with and provides commands to the radiation source controller 136, the C-arm motor controller 138, and the DAS 140 for controlling system operations such as data acquisition and/or data processing. In some embodiments, the computing device 144 controls operation of the radiation source controller 136, the C-arm motor controller 138, and the DAS 140 based on a user input.

Computing device 144 also includes a window generating module 160, similar to that disclosed in US Patent No. 9,349,199, entitled *System And Method For Generating Image Window View Settings*, the entirety of which is expressly incorporated herein by reference in its entirety for all purposes, that is configured to receive an image dataset, such as a 2D projection image dataset/transmission dataset 162, from the detector 120 and to implement various methods described herein. For example, the window generating module 160 may be configured to generate a viewing window having a predetermined window level and predetermined window width that are automatically determined based on the subject being viewed. The window generating module 160 may be implemented as a piece of hardware that is installed in the processor 146. Optionally, the window generating module 160 may be implemented as a set of instructions that are installed on the processor 146. The set of instructions may be stand-alone programs, may be incorporated as subroutines in an operating system installed on the processor 146, and/or in system memory 148 to be accessed by the processor 146, may be functions that are installed in a software package on the processor 146, or may be a combination of software and hardware. It should be understood that the various embodiments are not limited to the arrangements and instrumentality shown in the drawings.

Looking now at FIG. 4, a flowchart of a method 1000 employed by the medical radiography imaging system 100 and the window generating module 160 is illustrated for reconstructing an image of an object/subject 118 from using various inputs to the window generating module 160, such as the 2D projection dataset, in accordance with various embodiments. The method 1000 may be implemented as a set of instructions on the processor window generating module 160 and/or the processor 146 both shown in FIG. 3. More specifically, the method 1000 may be provided as a non-transitory machine-readable medium or media having instructions recorded thereon or an algorithm or artificial intelligence for directing the processor 146 or the window generating module 160 to perform an embodiment of the method described herein. The medium or media may be any type of CD-ROM, DVD, floppy disk, hard disk, optical disk, flash RAM drive, or other type of computer-readable medium or a combination thereof.

The method 1000 automatically generates a set of viewing parameters that are then automatically applied to an image to be displayed. The set of viewing parameters may include a window level setting and a window width setting. A window level setting is defined by a single pixel density value that is preselected based on a region or type of tissue of interest for presentation on the display 150. In one embodiment, the region or tissue of interest is automatically selected without user interaction. Optionally, the region of interest may be automatically selected in conjunction with certain manual inputs employed within the automatic selection performed by the window generating module 160.

Referring again to FIG. 4, at 1004, the subject 118 is scanned to generate a 2D projection dataset/transmission dataset, such as for example, the transmission dataset 162 shown in FIG. 1, also referred to herein as CT projection data/projection images, with each projection/image obtained at a different angle and/or position of the C-arm 102 relative to the subject 118. It should be realized that although the method 1000 is described with respect to the transmission dataset 162 obtained from the medical radiography imaging device or system 100, the method 1000 may also be applied to a transmission dataset obtained from a medical radiography imaging system forming a part of a dual-modality imaging system. Moreover, the method 1000 may be applied to any image dataset obtained using any of the imaging modalities discussed herein and the transmission dataset 162 is exemplary only. Accordingly, in various embodiments, the transmission dataset 162 is obtained using the medical radiography imaging system 100 (shown in FIG. 1). The transmission dataset 162 may be obtained by performing scan of the subject 118 to produce the transmission dataset 162. In various other embodiments, the transmission dataset 162 may be obtained from data collected during a previous scan of the subject 118, wherein the transmission dataset 162 has been stored in a memory, such as system memory 148. The transmission dataset 162 may be obtained during real-time scanning of the subject 118. For example, the methods described herein may be performed on transmission data as the transmission data is received from the detector 122 during a real-time examination of the subject 118.

At 1006, the 2D projection dataset/transmission dataset 162 is used by the processor 146 to generate a 3D volume/3D volume dataset 164 utilizing known computed tomography/tomographic processing methods. Within this 3D volume dataset 164 are a created a number of slices 166, in one embodiment formed with equal thicknesses, that collectively form the 3D volume dataset 164 representing the imaged volume or portion of the subject 118.

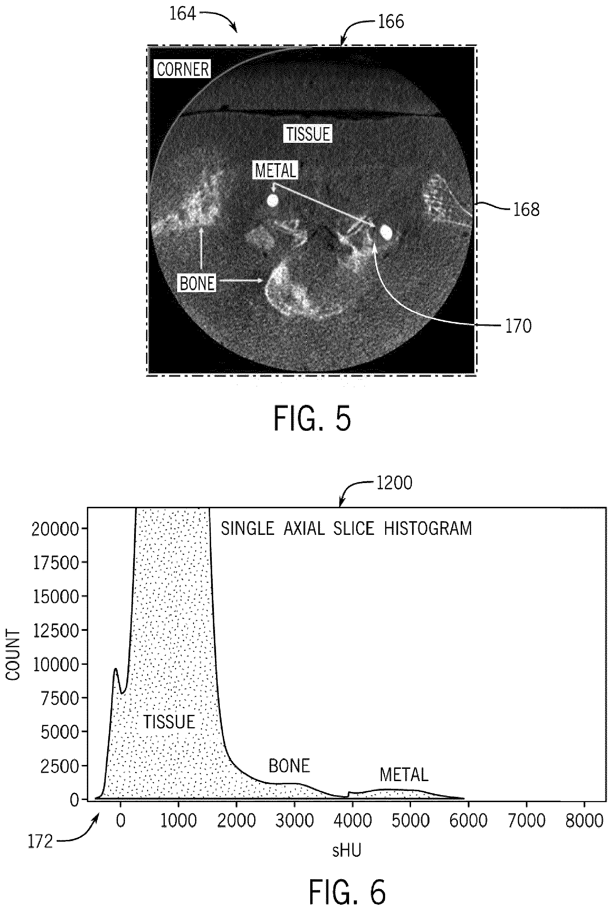

At 1008, the processor 146 is operated to determine a central slice 168 (FIG. 5) within the 3D volume dataset 164. The central slice 168 can be selected as the slice 166 disposed at or adjacent to the center of the 3D volume/3D volume dataset 164. In an alternative embodiment, the central slice 168 can be selected in any other suitable manner, such as by combining one or more slices 166 disposed around the center of the 3D volume/3D volume dataset 164, or by selecting one or more slices 166 disposed at or adjacent to an object of interest 170 identified within the 3D volume/3D volume dataset 164. Further, in other exemplary embodiment more than one central slice 168 can be selected, with each central slice 168 corresponding to an associated object of interest within the 3D volume/3D volume dataset 164. Any selected object of interest 170 may be identified automatically, such as by a computer-aided detection (CAD) system (not shown) operably connected to the processor 146, and/or by the user by manually positioning a marker that identifies the location of the object 170 within the 3D volume/3D volume dataset 164, for example.

At 1010, the window generating module 160 and/or the processor 146 containing the window generating module 160, performs a volume content analysis 172 on the central slice 168 in order to determine the distribution of the content, e.g., the material and/or tissue types, represented within the volume defined by the central slice 168. In performing this determination, the window generating module 160 analyzes the distribution of the Hounsfield units (HUs) for each pixels across the central slice 168 to generate a histogram 1200 (FIG. 6) for the central slice 168. Alternatively, the histogram 1200 can be generated using the HUs for selected and/or multiple slice 168, and/or across the entire 3D volume 164. The histogram 1200 provides an indication of the types and relative amounts of materials present within the central slice 168 as a result of the range and number of HUs detected for each of the pixels/voxels within the central slice 168.

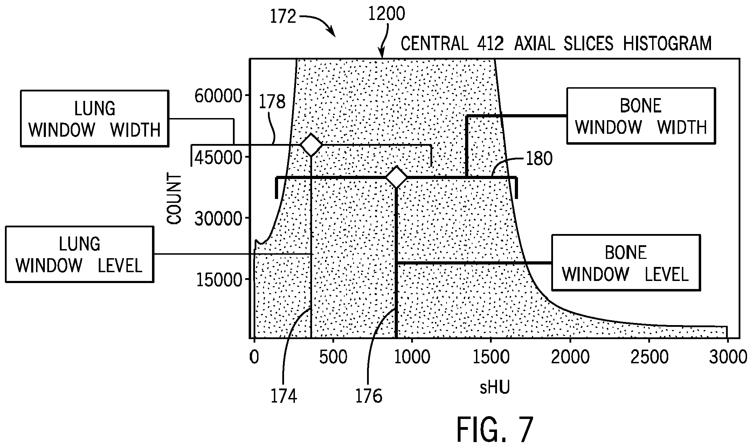

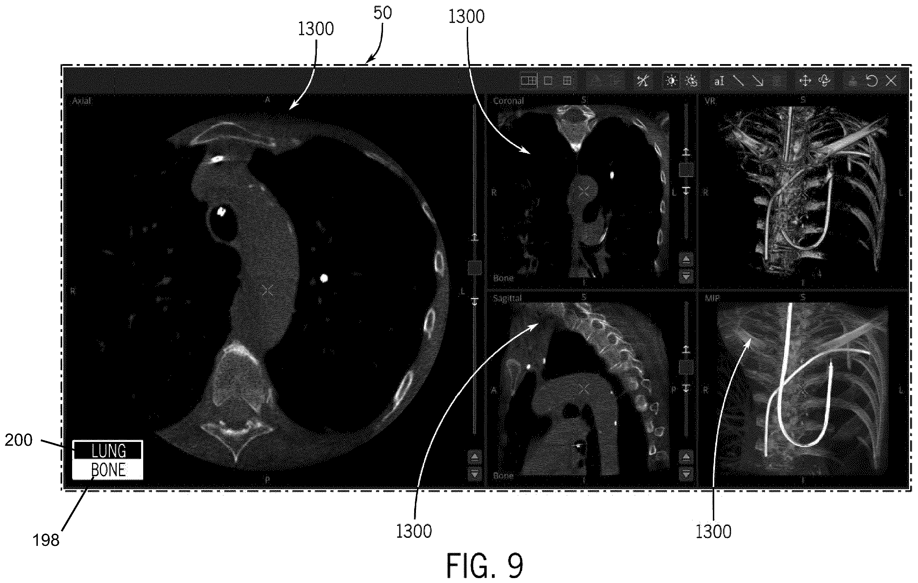

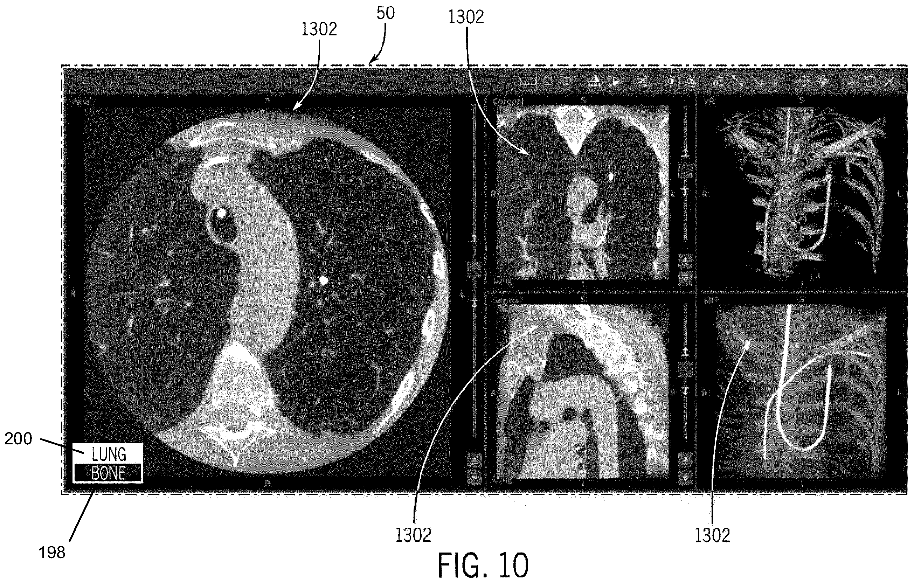

Referring now to FIGS. 4 and 7, at 1012 using the results of the volume content analysis 172 graphically represented in the histogram 1200, though it is to be understood that the results shown in the histogram 1200 may be represented within the window generating module 160 in other forms suitable for the performance of the method 1000 by the window generating module 160, such that histogram 120 can be omitted in its entirety, the window generating module 160 can determine a window level 174,176 for each of the material and/or tissue types present within the histogram 1200. Each window level 174,176 corresponds to a central grayscale value for a 2D image 1300,1302 (FIGS. 9 and 10) created using the 3D volume/3D volume dataset 164 that falls at or near the center of the range of HUs within which the type of material/tissue desired to be viewed is most readily discernable/has the highest contrast within the created 2D image 1300,1302. The window level 174,176 is determined automatically by the window generating module 160 as a result of the distribution of HUs present within the histogram 1200 and known information regarding predetermined values or ranges of values for window levels for different types of materials and/or tissues, and can optionally include manual input regarding starting values or ranges of values for the window levels for different types of materials and/or tissues.

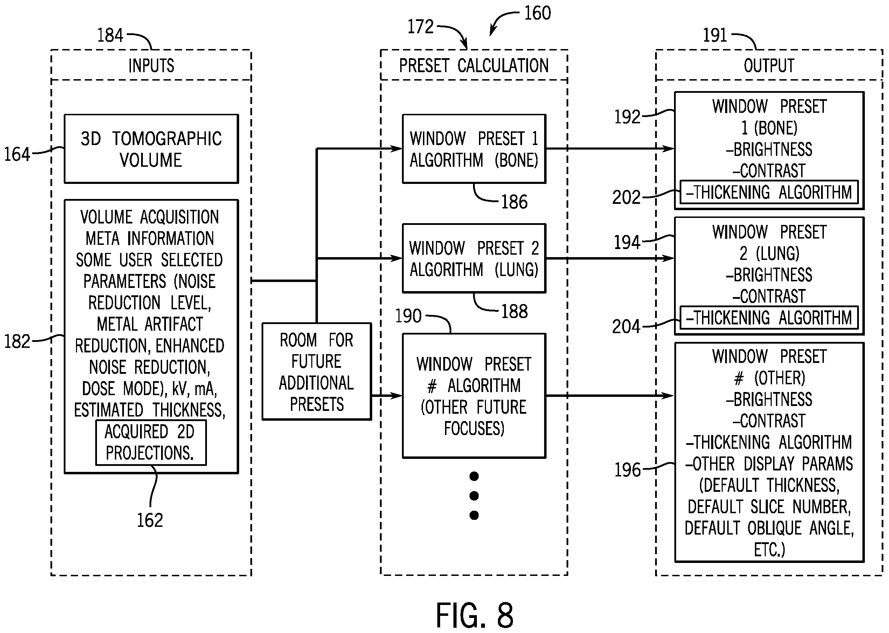

Referring now to FIG. 4 and 8, after determination of the window level 174,176 for the types of materials and/or tissues present in the central slice 168, at 1014 the window generating module 160 also determines a window width 178,180 around each corresponding window level 174,176 covering the range of HUs to optimally display the desired material and/or tissue type to be presented in the 2D image 1300,1302 in contrast to other materials and/or tissue types also present within the 2D image 1300,1302. The value of the window width 178,180 is defined by a range of pixel density values around the value for the window level 174,176. For example, the window generating module 160 may automatically generate a setting of the window width 178,180 of, for example, ±500 HU. Thus, if the setting for the window width 178,180 is automatically set to ±500 HU, the pixels in the image will be adjusted to have a range between +500 HU and -500 HU around the window level 174,176 for the HUs presented in the images 1300,1302. The value for the window width 178,180 can be automatically determined by the window generating module 160 based on the distribution of HUs present within the histogram 1200 and known information regarding predetermined values or ranges of values for window widths for different types of materials and/or tissues, and can optionally include manual input regarding starting values or ranges of values for the window widths for different types of materials and/or tissues.

Referring again to FIG. 4, in 1016 the window generating module 160 utilizes the window level 174,176 and the window width 178,180 to form window presets to be applied to 2D/3D images generated from the 3D volume/3D volume dataset 164 for optimized viewing of each type of material and/or tissue type present within the generated 2D/3D image(s) 1300,1302.

Referring now to FIG. 8, an exemplary embodiment of the method of operation of the window generating module 160 for determination of the window level(s) 174,176 and window width(s) 178,180 is illustrated. FIG. 8 additionally serves as an exemplary illustration of one training method for an AI system forming all or a part of the window generating module 160. In order to effectively determine the proper window level 174,176 (step 1012) and window width 178,180 (1014) for the highest contrast and viewability in a created 2D image 1300,1302 of the types of materials and/or tissues present in the central slice 168 according to the method of FIG. 4, the window generating module 160 initially receives the 3D volume/3D volume dataset 164, as well as any optional volume acquisition meta information 182 as inputs 184. The volume acquisition meta information 182 can include, but is not limited to, user-selected parameters for the acquisition of the 2D projection dataset, such as one or more or noise reduction level, metal artefact reduction, enhanced noise reduction, does mode, as well as the parameters for the operation of the radiation source, including but not limited to one or more of kV, mA, and estimated thickness of the subject 118, as well as the 2D projection dataset 162.

Utilizing these inputs 184, in performing the volume content analysis 172, the window generation module 160 employs one or more window or image preset algorithms 186,188,190 forming parts of the window generating module 160 and configured to determine the optimized viewing parameters for providing the highest contrast/best viewability for a particular type of material and/or tissue within one or more 2D images 1300,1302 to be generated from the 3D volume/3D volume dataset 164. For example, the window generation module 160 employs a bone preset algorithm 186 to analyze the inputs 184, e.g., the 3D volume/3D volume dataset 164 and any volume acquisition meta information 182, in the manner discussed previously to determine the central slice(s) 168, the histogram 1200 for the central slice 168 and the associated window level 174 and window width 178 in order to provide a corresponding 2D image 1300 that provides the optimal contrast for viewing bone within the image(s) 1300. In addition, the window generation module 160 can also employ a lung image preset algorithm 188, as well as one or more additional window or image preset algorithms 190, to similarly utilize the analysis of the inputs 184 to generate a window level 176 and window width 180 corresponding to the lungs, or other material and/or tissue types in order to provide corresponding 2D image(s) 1302 that provides the optimal contrast for viewing the lungs or other material/tissue within the image(s) 1302.

The outputs 191 from each of the window or image preset algorithms 186,188,190 can be provided to the processor 146 as a window preset 192,194,196 that can be stored on the system 100, such as in memory 148, for access and use by the processor 146 in presenting an image corresponding to the type of material and/or tissue associated with the preset algorithm 186,188,190. For example, the bone preset algorithm 186 outputs a bone window preset 192 that is employed by the processor 146 to present an image 1300 on the display 50 with the window level 174 and window width 178, as well as any other image parameters determined by the preset algorithm 186, to provide the image 1300 with the optimal brightness and contrast for representation of bone within the image 1300. The other window presets 194,196 contain information concerning the window level 176 and window width 180, as well as any other image parameters determined by the preset algorithm(s) 188,190, to provide the image 1300 with the optimal brightness and contrast for representation of the other materials and/or tissues, e.g., the lungs, within the image 1300. In one embodiment of the method 1000, the bone window preset 192 is employed by the processor 146 as a default setting for an image 1300 presented on the display 50.

According to another embodiment of the disclosure, as best shown in FIGS. 9 and 10, the additional window presets 194,196 can be presented on the display 50 in conjunction with the image(s) 1300,1302. The preset(s) 194,196 take the form of one or more icons 198,200 located on the display adjacent the image(s) 1300,1302. The one or more icons 198,200 are selectable by the user through the operation of the user interface 152 and serve to provide an instruction to the processor 146 to alter the parameters for the presentation of the image(s) 1300,1302 on the display 50. For example, the selection of the window preset 194 associated with the lungs causes the processor 146 to apply the window level 176 and the window width 178 to the image(s) 1300, resulting in the switch to the presentation of image(s) 1302 on the display 50 that utilizes the optimal brightness and contrast for representation of the lungs, within the image(s) 1302. In this manner, the user can quickly switch or toggle the image(s) 1300,1302 presented on the display 50 between views having the optimal brightness and contrast for representation of selected materials and/or tissues within the image(s) 1300,1302, e.g., between an image(s) 1300 optimized for viewing bone and an image(s) 1302 optimized for viewing lung tissue, without having to manually determine the window levels and window widths for each different material and/or tissue type.

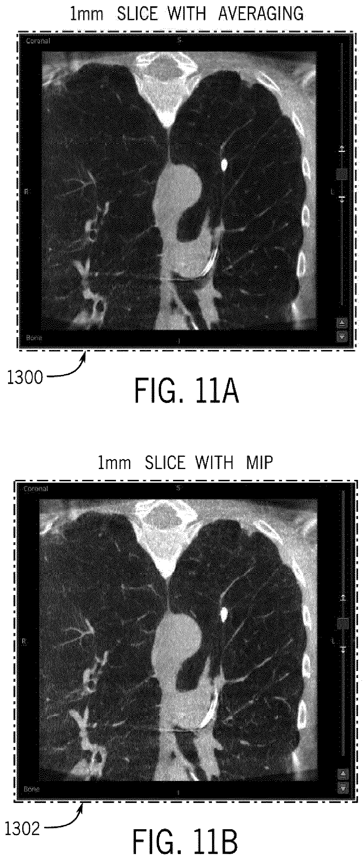

In addition, with regard to the information present in the window presets 192,194,196 output by the respective preset algorithms 186,188,190, the information can include a thickening algorithm 202,204,206 to be employed with regard to the type or material and/or tissue associated with the window preset 192,194,196. The thickening algorithm 202,204,206 can an algorithm that provides an averaged value for the brightness of the pixels across the 3D volume/3D volume dataset 164 in the presentation of the image(s) 1300, such as for the bone window preset 192, as shown in FIG. 11A. However, an algorithm 204 associated with the lung window preset 194 can utilize a maximum intensity projection (MIP) calculation for the 3D volume/3D volume dataset 164 to present the image(s) 1302 for the for the lung window preset 194 with pixels/voxels having the highest intensity values across the entire 3D volume/3D volume dataset 164, as shown in FIG. 11B. With this thickness algorithm 204 applied to the image 1302 produced using the lung window preset 194, the image(s) 1302 significantly enhances the viewability of small lung airways and/or nodules in the image(s) 1302, in comparison with the image(s) 1300 utilizing an averaged intensity algorithm 202.

Further, the method 1000 of the window generating module 160 can be employed separately for each imaging procedure performed by the medical radiography imaging device 100 to accommodate for the different amounts of materials and/or tissue types present within the 3D volumes/3Dvolue datasets of the subject of the particular imaging procedure.

The written description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims.

## Claims

1. A method for adjusting a presentation of an image presented on a display (148) of a radiography imaging system100), the method comprising the steps of:
a. providing a radiography imaging system (100) comprising:
i. a radiation source (120);
ii. a detector (122) alignable with the radiation source (120), the detector (122) having a support (116) on or against which a subject (118) to be imaged is adapted to be positioned;
iii. a computing device (144) operably connected to the detector (122) to generate image data in an imaging procedure performed by the imaging system (100), the computing device (144) including a processor (146) and an interconnected database (148) containing machine-readable instructions for the operation of the processor (146) and for processing the image data from the detector (122) to create one or more 2D images of a subject;
iv. a display (150) operably connected to the computing device (144) for presenting the one or more 2D images to a user; and
v. a user interface (152) operably connected to the computing device (144) to enable user input to a control processing unit (146);
b. positioning the subject (118) between the radiation source (120) and the detector (122);
c. operating the radiation source (120) to generate a plurality of projection images of the subject (118);
d. reconstructing a 3D volume (164) from the plurality of projection images;
e. determining a distribution of radiation attenuation values (172) from at least one portion of the 3D volume (164); and
f. determining a window preset (192,194) from the distribution of radiation attenuation values (172) corresponding to each type of material represented in the distribution of radiation attenuation values (172).

2. The method of claim 1, wherein the processor (144) includes a window generating module (160), and wherein the window generating module (160) is operable to:
a. select the at least one portion (166) of the 3D volume (164);
b. determine the distribution of radiation attenuation values (164) across the selected at least one portion (166) of the 3D volume (164);
c. determine a first window level (174) and a first window width (178) for a first material type represented in the distribution of radiation attenuation values (172) to form a first window preset (192); and
d. determine a second window level (176) and a second window width (180) for a second material type represented in the distribution of radiation attenuation values (172) to form a second window preset (194).

3. The method of claim 2, wherein the window generating module (160) is formed at least partially of an artificial intelligence.

4. The method of claim 2, wherein the window generating module (160) is operable to include a first thickening algorithm (202) for the one or more 2D images with the first window preset (192) and a second thickening algorithm (204) for the one or more 2D images with the second window preset (194).

5. The method of claim 1, further comprising the step of presenting the window image preset (192,194) as a selectable icon (198,200) on the display (150).

6. The method of claim 1, wherein the step of determining the distribution of radiation attenuation values (172) comprises:
a. selecting the at least one portion (166) of the 3D volume (164);
b. determining the distribution of radiation attenuation values (172) across the selected at least one portion (166) of the 3D volume (164); and
c. determining a first window level (174) and a first window width (178) for a first material type represented in the distribution of radiation attenuation values (172) to form a first window preset (192).

7. The method of claim 6, further comprising the step of:
a. determining a second window level (176) and a second window width (180) for the second material type represented in the distribution of radiation attenuation values (172) to form a second window preset (194).

8. The method of claim 7, further comprising the step of applying the first window preset (192) as a default window preset for the one or more 2D images presented on the display (150).

9. The method of claim 7, further comprising the steps of:
a. applying a first thickening algorithm (202) for the one or more 2D images with the first window preset (192); and
b. applying a second thickening algorithm (204) for the one or more 2D images with the second window preset (194).

10. The method of claim 9, wherein the first window preset (192) is a lung window preset and the first thickening algorithm (202) is a maximum intensity projection thickening algorithm.

11. The method of claim 6, wherein the step of selecting the at least one portion (166) of the 3D volume (164) comprises selecting a central portion (166) of the 3D volume (164).

12. The method of claim 9, wherein the 3D volume (164) includes a number of slices (166), and wherein the step of selecting a central portion (166) of the 3D volume (164) comprises selecting a central slice (166) of the 3D volume (164).

13. The method of claim 1, wherein the radiography imaging system (100) is a C-arm radiography imaging system including a base (104) and a C-arm (102) movably connected to the base (104), the C-arm (102) including the radiation source (120) and the detector (122) disposed thereon, and wherein the step of operating the radiation source (120) to generate a plurality of projection images of the subject (118) comprises moving the C-arm (102) to position the radiation source (120) and the detector (122) at a number of angular positions relative to the subject (118).

14. A radiography imaging device (100) comprising:
a. a radiation source (120);
b. a detector (122) alignable with the radiation source (122), the detector (120) having a support (122) on or against which a subject (118) to be imaged is adapted to be positioned;
c. a computing device (144) operably connected to the detector (122) to generate image data in an imaging procedure performed by the radiography imaging device (100), the computing device (144) including a processor (146) and an interconnected database (148) containing machine-readable instructions for the operation of the processor (146) and for processing the image data from the detector (120) to create one or more 2D images of the subject (118) and to reconstruct a 3D volume (164) from the one or more 2D images;
d. a display (150) operably connected to the computing device (148) for presenting the one or more 2D images, the 3D volume (164) or one or more portions (166) thereof, and combinations thereof to a user; and
e. a user interface (152) operably connected to the computing device (144) to enable user input to the control processing unit (146),
wherein the processor (146) is configured to determine the distribution of radiation attenuation values (172) across at least one portion (166) of the 3D volume (164), to determine a first window level (174) and a first window width (178) for a first material type represented in the distribution of radiation attenuation values (172) to form a first window preset (192); and to determine a second window level (176) and a second window width (180) for a second material type represented in the distribution of radiation attenuation values (172) to form a second window preset (194).

15. The radiography imaging device (100) of claim 14, wherein the processor (146) is configured to apply the first window preset (192) as a default window preset for the one or more 2D images presented on the display (150) and to present the second window preset (194) as a selectable icon (198,200) on the display (150) in association with the one or more 2D images.
